(19) Europäisches Patentamt
European Patent Office
Office européen des brevets

(11) Veröffentlichungsnummer : **0 473 552 A1**

(12) # EUROPÄISCHE PATENTANMELDUNG

(21) Anmeldenummer : **91810674.1**

(51) Int. Cl.[5] : **C07C 209/36**

(22) Anmeldetag : **23.08.91**

(30) Priorität : **31.08.90 CH 2828/90**

(43) Veröffentlichungstag der Anmeldung : **04.03.92 Patentblatt 92/10**

(84) Benannte Vertragsstaaten : **CH DE FR GB IT LI NL**

(71) Anmelder : **CIBA-GEIGY AG**
**Klybeckstrasse 141**
**CH-4002 Basel (CH)**

(72) Erfinder : **Baumeister, Peter**
**St. Annaweg 24**
**CH-4113 Flüh (CH)**
Erfinder : **Scherrer, Wilfried**
**Feldstrasse 10**
**CH-4416 Bubendorf (CH)**

(54) **Verfahren zur Herstellung von halogenierten aromatischen primären Aminen.**

(57) Die katalytische Hydrierung von halogenierten Nitroaromaten mit Edelmetallkatalysatoren in Gegenwart eines Formamidinsalzes als Dehalogenierungsinhibitor liefert innerhalb kurzer Reaktionszeiten auch bei erhöhten Temperaturen halogenierte aromatische primäre Amine in hohen Ausbeuten und hoher chemischer Reinheit.

EP 0 473 552 A1

Die Erfindung betrifft ein Verfahren zur Herstellung von halogenierten aromatischen primären Aminen durch katalytische Hydrierung von halogenierten aromatischen Nitroverbindungen in Gegenwart von Edelmetallkatalysatoren und einem Formanlidinsalz als Dehalogenierungsinhibitor.

Es ist bekannt, dass man durch katalytische Hydrierung von halogenierten, aromatischen Nitroverbindungen halogenierte aromatische primäre Amine erhält. Die Hydrierung führt auch zu einer Dehalogenierung und damit zu schwer trennbaren Gemischen halogenfreier und halogenierter aromatischer primärer Amine. Es sind verschiedene Vorschläge gemacht worden, die Dehalogenierung zu inhibieren.

In der US-PS 3 361 819 wird bei der katalytischen Hydrierung von Halogennitroaromaten mit Edelmetallkatalysatoren der Zusatz von Aminen, z.B. Morpholin oder N-Methylmorpholin als Inhibitoren empfohlen. In der US-PS 4 070 401 wird vorgeschlagen, bei der gleichen Reaktion offenkettige Amine, z.B. Isopropylamin oder Triethylentetramin als Inhibitor einzusetzen. Ferner wird in der US-PS 4 070 401 empfohlen, Schwefelverbindungen wie z.B. Thioether, Thiarane oder Dithiane als Inhibitoren zu verwenden.

In der EP-A-0 325 892 wird ein Verfahren zur Hydrierung von Halogennitroaromaten in Gegenwart von Raney-Nickel beschrieben, bei dem Formanmidinsalze als wirksame Dehalogenierungsinhibitoren verwendet werden. Die Verwendung von Raney-Nickel hat den Nachteil, dass das Reaktionsprodukt stets mit unerwünschten löslichen Nickelsalzen kontaminiert ist.

Es wurde nun gefunden, dass Formamidinsalze die Dehalogenierung auch bei der Hydrierung von halogenierten aromatischen Nitroverbindungen in Gegenwart von Edelmetallkatalysatoren wirksam unterdrücken. Die gewünschten halogenierten aromatischen Amine werden unter günstigen Bedingungen sogar in praktisch quantitativen Ausbeuten erhalten, wobei der Gehalt an Nebenprodukten bei optimalen Bedingungen wesentlich unter 1 Gew.-% beaagen kann. Die Katalysatoraktivität wird nicht beeinträchrigt und die Hydriergeschwindigkeit wird nicht nennenswert beeinflusst. Im Gegensatz zur Verwendung von Raney-Nickel-Katalysatoren wird überraschend keine Korrosion der Edelmetallkatalysatoren beobachtet und die Reaktionsgemische enthalten keine messbaren Anteile an löslichen Edelmetallsalzen. Das Verfahren ist zur Durchführung im grosstechnischen Massstab geeignet.

Gegenstand der Erfindung ist ein Verfahren zur Herstellung von halogenierten aromatischen primären Aminen durch katalytische Hydrierung von halogenierten aromarischen Nitroverbindungen in Gegenwart eines Edelmetallkatalysators bei einem Druck von 0,1 bis 100 bar und bei einer Temperatur von 30 bis 150°C in einem inerten Lösungsmittel und in Gegenwart eines Inhibitors gegen eine Dehalogenierung, das dadurch gekennzeichnet ist, dass der Inhibitor ein Formamidinsalz ist.

Als Nitroverbindungen kommen z.B. solche der allgemeinen Formel

$$(Y)_x\text{—}A\text{—}(NO_2)_y$$

in Frage, worin x und y unabhängig voneinander eine Zahl von 1 bis 6, bevorzugt 1 bis 3 und besonders bevorzugt 1 oder 2 bedeuten, Y für Halogen, bevorzugt für F, Cl und Br,steht, und A einen aromatischen Rest mit 6-18 C-Atomen darstellt, der noch weitere Substituenten enthalten kann. Y bedeutet bevorzugt Cl oder Br und besonders bevorzugt Cl. In einer besonders bevorzugten Ausführungsform stehen Y für Cl, x für 1 oder 2 und y für 1, 2 oder 3.

Der aromatische Rest enthält bevorzugt 6 bis 14, besonders 6-10 C-Atome und stellt insbesondere einen aromatischen Kohlenwasserstoffrest dar. Beispiele für aromatische Kohlenwasserstoffe, von denen sich der Rest ableiten kann, sind zum Beispiel: Benzol, Naphthalin, Phenanthren, Biphenyl, Indan, durch hydrierstabile Brückengruppen $Y_1$ verknüpftes Benzol, wie z.B.

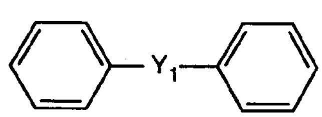
,

worin $Y_1$ $C_1$-$C_4$-Alkylen, $C_1$-$C_6$-Alkyliden, -O-, -S-, -$SO_2$- oder -CO- sein kann.

Geeignete weitere Substituenten für den Rest A sind z.B. $C_1$-$C_4$-Alkyl, -Halogenalkyl, -Alkoxy, -Halogenalkoxy, oder -Alkylthio, $C_5$-oder $C_6$-Cycloalkyl, Cyano, Hydroxyl, $C_1$-$C_8$-Acyl oder -Acyloxy, -COOH, -$SO_3$H, -COOM und -$SO_3$M, worin M für ein Alkalimetallkarion, z.B. Na⊕, oder Ammonium steht, -$SO_3R_3$ oder -$CO_2R_3$, worin $R_3$ $C_1$-$C_6$-Alkyl, Phenyl oder Benzyl ist, -$NH_2$, $C_1$-$C_8$-Alkyl- oder -Dialkylamino, $C_1$-$C_8$-Acylamino oder $C_1$-$C_8$-Aminocarbonyl. Einige Beispiele sind Methyl, Ethyl, n-oder iso-Propyl, n-, i- oder t-Butyl, Methyloxy, Ethyloxy, Chlorethyloxy, Methylthio, Chlormethyl, Fluormethyl, Trifluormethyl, Trichlormethyl, Cyclopentyl, Cyclohexyl, Acetyl, Propionyl, Acetyloxy, Methoxysulfonyl, Methoxy- oder Ethoxycarbonyl, Methylamino, Dimethylamino, Acetylamino, Aminocarbonyl und Dimethylaminocarbonyl.

Beispiele für Nitroverbindungen sind o-, m- und p-Chlor- oder -Bromnitrobenzol, 2,3-, 3,4-, 2,4-, 2,6-, 3,5- und 2,5-Dichlor- oder -Dibromnitrobenzol, 2,3,4- und 2,3,5-Trichlornitrobenzol, 2-Chlor-3-Bromnitrobenzol, 1 -Chlor- oder 1 -Brom-2,4-dinitrobenzol, 3,4-Dichlor-1,6-dinitrobenzol, 2,4-Dichlor- 1,6-dinitrobenzol, 1 -Chlor-2,4,6-trinitrobenzol, 4-oder 6-Chlor-1-methyl-2-nitrobenzol, 2- oder 6-Brom-1-methyl-4-nitrobenzol, 4- oder 6-Chlor-1-ethyl-2-nitrobenzol, 2- oder 3-Brom-1-ethyl-4-nitrobenzol, 4- oder 6-Chlor-1-methoxy-4-nitrobenzol, 4- oder 6-Chlor-2-nitrophenol, 1-Chlor- oder 1-Brom-2-nitronaphthalin, 1-Chlor- oder 1-Brom-2,7-dinitronaphthalin, 2-Chlor-4-nitrodiphenyl, 2,2′-Dichlor-4,4′-dinitro-diphenyl, 2-Brom-4,4′-dinitrobenzophenon, Bis(3,3′-dichlor-4,4′-dinitrophenyl)-methan und 3,3′-Dichlor-4,4′-dinitrodiphenylether. Bevorzugt sind Mono- und Dichlornitroverbindungen mit 1 bis 3, bevorzugt 1 bis 2 Nitrogruppen. Besonders bevorzugt ist 1-Chlor-2,4-dinitrobenzol.

Das Formamidinsalz wird vorteilhaft in einer Menge von 0,1 bis 30 Mol-%, bevorzugt 0,1 bis 20 Mol-% und besonders 0,5 bis 15 Mol-% verwendet, bezogen auf die Nitroverbindung.

Das Formamidinkation kann mit einem, zwei oder drei Kohlenwasserstoffresten substituiert sein, die vorzugsweise 1 bis 18, besonders 1-12, und insbesondere 1 bis 6 C-Atome enthalten. Das Anion des Formamidinsalzes kann sich z.B. von aliphatischen oder aromatischen Carbonsäuren, bevorzugt Mono- oder Dicarbonsäuren ableiten, die vorzugsweise 1 bis 18, besonders 1-8, und insbesondere 1 bis 4 C-Atome enthalten.

Eine bevorzugte Ausführungsform ist jene, worin das Formamidinsalz der Formel I

$$[R_3N=\overset{H}{\overset{|}{C}}-\overset{H}{\overset{|}{N}}R_1R_2]_n^{\oplus}X^{n\ominus} \qquad (I)$$

entspricht, worin $R_1$, $R_2$ und $R_3$ unabhängig voneinander für H stehen, oder lineares oder verzweigtes $C_1$-$C_{12}$-Alkyl, $C_5$- oder $C_6$-Cycloalkyl, $C_6$-$C_{18}$-Alkylcycloalkyl, $C_6$-$C_{10}$-Cycloalkylalkyl, $C_7$-$C_{18}$-Alkylcycloalkylalkyl, wobei das Cycloalkyl 5 oder 6 Ring-C-Atome enthält, $C_6$-$C_{10}$-Aryl, $C_7$-$C_{18}$-Alkaryl, $C_7$-$C_{12}$-Aralkyl oder $C_7$-$C_{18}$-Alkaralkyl oder $R_1$ und $R_2$ zusammen Tetra- oder Pentamethylen oder 3-Oxa-1,5-Pentylen darstellen, X das Anion einer Säure, besonders einer $C_1$-$C_{18}$-Mono- oder -Dicarbonsäure bedeutet, und n 1 oder 2 ist.

$R_1$, $R_2$ und $R_3$ enthalten als Alkyl vorzugsweise 1 bis 6 und besonders 1 bis 4 C-Atome. Beispiele sind Methyl, Ethyl und die Isomeren von Propyl, Butyl, Pentyl, Hexyl, Heptyl, Octyl, Decyl und Dodecyl. $R_1$, $R_2$ und $R_3$ sind als Cycloalkyl z.B. Cyclopentyl und Cyclohexyl. Als Alkylcycloalkyl enthalten $R_1$, $R_2$ und $R_3$ bevorzugt 6 bis 12, besonders 6 bis 10 C-Atome. Beispiele sind Methylcyclopentyl, Methylcyclohexyl, Ethylcyclohexyl und n-Propylcyclohexyl. $R_1$, $R_2$ und $R_3$ sind als Cycloalkylalkyl besonders Cyclopentylmethyl oder Cyclohexylmethyl. Als Alkylcycloalkylalkyl enthalten $R_1$, $R_2$ und $R_3$ bevorzugt 7 bis 12 C-Atome und sind insbesondere $C_1$-$C_4$-Alkylcyclopentylmethyl oder -cyclohexylmethyl. $R_1$, $R_2$ und $R_3$ in der Bedeutung von Aryl kann z.B. Naphthyl und besonders Phenyl sein. In den Aryl enthaltenden Resten für $R_1$, $R_2$ und $R_3$ ist das Aryl besonders Phenyl. $R_1$, $R_2$ und $R_3$ in der Bedeutung von Alkaryl enthalten bevorzugt 7 bis 12 C-Atome und stellen besonders $C_1$-$C_4$-Alkylphenyl dar. $R_1$, $R_2$ und $R_3$ als Aralkyl sind besonders Benzyl und Phenylethyl. $R_1$, $R_2$ und $R_3$ als Alkaralkyl enthalten bevorzugt 8 bis 14 C-Atome und stellen besonders $C_1$-$C_4$-Alkylbenzyl dar.

Bei $R_1$, $R_2$ und $R_3$ handelt es sich bevorzugt um H oder aliphatische beziehungsweise cycloaliphatische Reste.

In einer bevorzugten Ausführungsform stellen $R_1$, $R_2$ und $R_3$ unabhängig voneinander H oder $C_1$-$C_6$-Alkyl, besonders H, Methyl oder Ethyl und insbesondere jeweils H dar.

In Formel I steht n vorzugsweise für 1.

X als Anion einer Mono- oder Dicarbonsäure enthält bevorzugt 1-18, besonders 1-12 und insbesondere 1 bis 8 C-Atome. Es kann sich um aliphatische oder aromatische Carbonsäuren handeln, die z.B. den Formeln

$$R_4COOH \text{ oder } R_5(COOH)_2$$

entsprechen können, worin $R_4$ lineares oder verzweigtes Alkyl mit besonders I bis 6 C-A-tomen, Cyclohexyl, Phenyl oder Benzyl ist, und $R_5$ eine direkte Bindung, lineares oder verzweigtes $C_2$-$C_6$-Alkylen, Cyclohexylen oder Phenylen darstellt. Insbesondere stellt X in Formel I das Anion einer aliphatischen Mono-oder Dicarbonsäure mit bevorzugt 1 bis 4 C-Atomen dar. Besonders steht X für die Anionen $(COO)_2^{2\ominus}$, $HCOO^{\ominus}$, $CH_3COO^{\ominus}$, $CH_3CH_2COO^{\ominus}$, $CH_3CH_2CH_2COO^{\ominus}$ oder $CH_2(COO)_2^{2\ominus}$ und ganz besonders für $CH_3COO^{\ominus}$.

Insbesondere handelt es sich bei dem Formanlidinsalz um unsubstituierte Formanlidinsalze, ganz besonders um Formanlidinacetat.

Das Formamidinsalz kann als solches dem Reaktionsgemisch beigegeben werden, oder in bekannter

Weise vor der Hydrierung in situ erzeugt werden. Die in situ Bildung des gegebenenfalls substituierten Formamidinsalzes erfolgt so schnell, dass man diese Reaktion in Gegenwart einer halogenierten aromatischen Nitroverbindung durchführen kann. Es kann hierbei ein Teil der Nitroverbindung zugegeben und der Rest nachdosiert werden, oder es kann die gesamte Menge Nitroverbindung mitvorgelegt werden.

Die Menge an Edelmetallkatalysator beträgt bevorzugt 0,1 bis 10 Gew.-%, besonders 0,1 bis 5 Gew.-%, bezogen auf die Nitroverbindung.

Geeignete Edelmetalle sind zum Beispiel solche aus der Platingruppe, besonders Platin oder Rhodium. Besonders bevorzugt ist Platin. Die Edelmetalle werden im allgemeinen auf einem Trägermaterial aufgebracht, z.B. feinteiliger Kohle oder Aluminiumoxid, z.B. in Mengen von 1 bis 10 Gew.-%, bezogen auf das Trägermaterial. Bevorzugter Katalysator ist Platin, das auf feinteiliger Kohle oder Aluminiumoxid aufgebracht ist, bevorzugt auf feinteiliger Kohle.

Die Hydrierung wird in Substanz oder in einem inerten Lösungsmittel durchgeführt. Geeignete Lösungsmittel sind z.B. Alkanole (Methanol, Ethanol, Propanol, Butanol, Methoxy- oder Ethoxyethanol), Ether (Dibutylether, t-Butylmethylether, Tetrahydrofuran, Dioxan, Ethylenglykoldimethylether, Diethylenglykoldimethylether), Amide und Lactame (Dimethylformanlid, Dimethylacetamid, N-Methylpyrrolidon), Ester und Lactone (Essigsäureethylester, γ-Butyrolacton), Kohlenwasserstoffe (Pentan, Hexan, Methylcyclohexan, Benzol, Toluol, Xylol, Chlorbenzol), Wasser. Bei der Hydrierung in Substanz ist das bei der Hydrierung entstehende halogenierte aromarische Amin das Lösungsmittel.

In einer bevorzugten Ausführungsform wird als Lösungsmittel ein $C_1$ -$C_4$-Alkanol alleine oder im Gemisch mit Wasser verwendet. Insbesondere wird Methanol verwendet.

Die Reaktionstemperatur beträgt vorteilhaft 50 bis 120°C. Der Druck beträgt vorzugsweise 1 bis 30 bar. Die Reaktionszeit richtet sich im wesentlichen nach den Reaktionsbedingungen und beträgt im allgemeinen weniger als zwei Stunden.

Das erfindungsgemässe Verfahren kann so durchgeführt werden, dass man in einem Autoklaven die Nitroverbindung, den Katalysator, das Lösungsmittel und das Formanlidinsalz einträgt und die Luft zuerst durch Stickstoff und diesen dann durch Wasserstoff verdrängt. Dann wird der Autoklav verschlossen, Wasserstoff bis zum gewünschten Druck aufgepresst und auf die Reaktionstemperatur erhitzt. Nach Beendigung der Reaktion wird das Reaktionsgemisch vom Katalysator abgetrennt. Danach kann man das Reaktionsprodukt vom Reaktionswasser und Lösungsmittel abtrennen und durch Destillation oder Umkristallisation weiter reinigen. Die primären aromatischen Amine sind bekannterweise Zwischenprodukte zur Herstellung von Farbstoffen.

Die nachfolgenden Beispiele erläutern die Erfindung näher.

Beispiel 1: 40,8 g 1-Chlor-2,4-dinitrobenzol, 0,21 g Pt/C (5 Gew.-%), 1,04 g Formamidinacetat und 120 ml Methanol werden in einem Autoklaven mit Begasungsrührer eingetragen. Anschliessend wird die Luft im Autoklaven durch Stickstoff und dann durch Wasserstoff verdrängt. Bei einem Druck von 10 bar und einer Temperatur von 60°C wird hydriert. Die Hydrierzeit beträgt 2,5 Stunden. Man erhält in quantitativer Ausbeute 1-Chlor-2,4-Diaminobenzol, das eine Reinheit von 98 % aufweist [flüssigchromatographische Analyse, (Nachweis als 1-Chlor-2,4-diacetamidobenzol)].

Beispiel 2: Es wird wie in Beispiel 1 verfahren, aber 0,21 g $Pt/Al_2O_3$ (5 Gew.-%) verwendet. Die Hydrierzeit beträgt 4,5 Stunden und die Reinheit des in quantitativer Ausbeute erhaltenen Produkts 96,6 %.

Beispiel 3: Es wird wie in Beispiel 1 verfahren, aber 0,42 g Rh/C (5 Gew.-%) verwendet. Die Reaktionstemperatur beträgt 80 °C und die Hydrierzeit beträgt 1,5 Stunden. Die Reinheit des in quantitativer Ausbeute erhaltenen Produkts beträgt 94,1 %.

## Patentansprüche

1. Verfahren zur Herstellung von halogenierten aromatischen primären Aminen durch katalytische Hydrierung von halogenierten aromatischen Nitroverbindungen in Gegenwart eines Edelmetallkatalysators bei einem Druck von 0,1 bis 100 bar und bei einer Temperatur von 30 bis 150°C in einem inerten Lösungsmittel und in Gegenwart eines Inhibitors gegen eine Dehalogenierung, dadurch gekennzeichnet, dass der Inhibitor ein Formamidinsalz ist.

2. Verfahren gemäss Anspruch 1, worin das Formamidinsalz in einer Menge von 0,1 bis 30 Mol-% verwendet wird, bezogen auf die Nitro-Verbindung.

3. Verfahren gemäss Anspruch 2, worin das Formamidinsalz in einer Menge von 0,5 bis 15 Mol-% verwendet wird.

**4.** Verfahren gemäss Anspruch 1, worin das Formamidinsalz der Formel I

$$[R_3N=\overset{H}{\overset{|}{C}}-\overset{H}{\overset{|}{N}}R_1R_2]_n^{\oplus}X^{n\ominus} \qquad (I)$$

entspricht, worin $R_1$, $R_2$ und $R_3$ unabhängig voneinander für H stehen, oder lineares oder verzweigtes $C_1$-$C_{12}$-Alkyl, $C_5$- oder $C_6$-Cycloalkyl, $C_6$-$C_{18}$-Alkylcycloalkyl, $C_6$-$C_{10}$-Cycloalkylalkyl, $C_7$-$C_{18}$-Alkylcycloalkylalkyl, wobei das Cycloalkyl 5 oder 6 Ring-C-Atome enthält, $C_6$-$C_{10}$-Aryl, $C_7$-$C_{18}$-Alkaryl, $C_7$-$C_{12}$-Aralkyl oder $C_7$-$C_{18}$-Alkaralkyl oder $R_1$ und $R_2$ zusammen Tetra- oder Pentamethylen oder 3-Oxa- 1,5-Pentylen darstellen, X das Anion einer Säure, besonders einer $C_1$-$C_{18}$-Mono- oder -Dicarbonsäure bedeutet und n 1 oder 2 ist.

**5.** Verfahren gemäss Anspruch 4, worin $R_1$, $R_2$ und $R_3$ unabhängig voneinander H, eine aliphatische oder cycloaliphatische Gruppe sind.

**6.** Verfahren gemäss Anspruch 4, worin in Formel I $R_1$, $R_2$ und $R_3$ unabhängig voneinander für H oder $C_1$-$C_6$-Alkyl stehen.

**7.** Verfahren gemäss Anspruch 4, worin in Formel I $R_1$, $R_2$ und $R_3$ unabhängig voneinander für H, Methyl oder Ethyl stehen.

**8.** Verfahren gemäss Anspruch 4, worin in Formel I $R_1$, $R_2$ und $R_3$ unabhängig voneinander je für H stehen.

**9.** Verfahren gemäss Anspruch 4, worin n in Formel I für 1 steht.

**10.** Verfahren gemäss Anspruch 4, worin X in Formel I als Anion einer Carbonsäure 1 bis 8 C-Atome enthält.

**11.** Verfaren gemäss Anspruch 4, worin X in Formel I das Anion einer a liphatischen $C_1$-$C_4$-Mono- oder Dicarbonsäure ist.

**12.** Verfahren gemäss Anspruch 11, worin X in Formel I $(COO)_2^{2\ominus}$, $HCOO^{\ominus}$, $CH_3COO^{\ominus}$, $CH_3CH_2COO^{\ominus}$, $CH_3CH_2CH_2COO^{\ominus}$ oder $CH_2(COO)_2^{2\ominus}$ ist.

**13.** Verfahren gemäss Anspruch 11, worin X für $CH_3COO^{\ominus}$ steht.

**14.** Verfahren gemäss Anspruch 1, worin das Formamidinsalz Formamidinacetat ist.

**15.** Verfahren gemäss Anspruch 1, worin der Edelmetallkatalysator in einer Menge von 0,1 bis 10 Gew.-% verwendet wird, bezogen auf die Nitroverbindung.

**16.** Verfahren gemäss Anspruch 1, worin als Lösungsmittel ein $C_1$-$C_4$-Alkanol alleine oder im Gemisch mit Wasser verwendet wird.

**17.** Verfahren gemäss Anspruch 16, worin das Lösungsmittel Methanol ist.

**18.** Verfaren gemäss Anspruch 1, worin die Temperatur 50 bis 120°C beträgt.

**19.** Verfahren gemäss Anspruch 1, worin der Druck 1 bis 30 bar beträgt.

**20.** Verfahren gemäss Anspruch 1, worin man das Formamidinsalz durch Hydrierung eines entsprechenden Cyanamids in Gegenwart einer Säure in situ herstellt, und die halogenierte aromatische Nitroverbindung entweder in der gesamten Menge mitvorlegt, oder teilweise mitvorlegt und den Rest nachdosiert.

**21.** Verfahren gemäss Anspruch 1, worin die Edelmetallkatalysatoren aus der Gruppe Platin und Rhodium ausgewählt sind, und die Metalle auf einem Trägermaterial aufgebracht sind.

**22.** Verfahren gemäss Anspruch 21, worin der Katalysator Platin ist, der auf feinteiliger Kohle oder feinteiligem Aluminiumoxid aufgebracht ist.

Europäisches Patentamt

# EUROPÄISCHER RECHERCHENBERICHT

Nummer der Anmeldung

EP 91810674.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,Y | EP - A - 0 325 892 (CIBA-GEIGY) * Patentansprüche 1-20 * | 1-22 | C 07 C 209/36 |
| D,Y | US - A - 4 070 401 (HIRAI) * Zusammenfassung; Spalte 3, Zeilen 57-63 * | 1-22 | |
| Y | DE - B - 2 549 900 (BAYER) * Spalte 1, Zeilen 40-60 * | 1-22 | |

RECHERCHIERTE SACHGEBIETE (Int Cl 5)

C 07 C 209/00

Der vorliegende Recherchenbericht wurde für alle Patentansprüche erstellt.

| Recherchenort | Abschlußdatum der Recherche | Prüfer |
|---|---|---|
| WIEN | 04-12-1991 | ONDER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82